(19) Europäisches Patentamt
European Patent Office
Office européen des brevets

(11) Publication number: 0 012 798 A1

(12) EUROPEAN PATENT APPLICATION

(21) Application number: 79103700.5

(22) Date of filing: 28.09.79

(51) Int. Cl.[3]: C 07 F 7/08
C 07 F 7/18, C 08 F 38/02
C 07 C 15/50, C 07 C 15/54
C 07 C 43/215, C 07 C 43/285
C 07 C 49/796, C 07 C 149/31
C 07 C 147/14

(30) Priority: 31.10.78 US 956517

(43) Date of publication of application: 09.07.80 Bulletin 80/14

(84) Designated Contracting States: DE FR GB IT

(71) Applicant: International Business Machines Corporation

Armonk, N.Y. 10504(US)

(72) Inventor: Economy, James
6694 Heathfield Drive
San Jose, California 95120(US)

(72) Inventor: Flandera, Mary Ann
312 Bromley Cross Drive
San Jose, California 95119(US)

(74) Representative: Chaudhry, Mohammad Saeed
IBM United Kingdom Patent Operations Hursley Park
Winchester Hants, S021 2JN(GB)

(54) **Polymerisable acetylenic oligomers and process for their preparation.**

(57) Soluble, polymerizable oligomers having a number average molecular weight of from about 200 to about 10,000, and having more than two pendant acetylenic groups.

The process for producing such oligomers: E.g. reacting phenyl triethynyl silane or triethynyl benzene in the presence of a catalyst with an aromatic acetylenic capping agent having a molar excess over the monomer.

EP 0 012 798 A1

TITLE MODIFIED
see front page



# POLYMERIZABLE ACETYLENIC OLIGOMERS

This invention relates to a polymerizable oligomer and a process of producing said oligomers.

U. S. patent 3,300,457 discloses the preparation of acetylenic polymers by the self-condensation reaction resulting from the oxidative coupling of organic compounds containing at least two terminal acetylenic groups directly bonded to the organic nucleus. The process disclosed in the U. S. Patent 3,300,457 comprises reacting such compounds with oxygen in homogeneous solution in the presence of a dissolved oxygen-carrying intermediate comprising an amine-basic cupric salt complex.

According to the invention there is provided a polymerizable oligomer having the general formula:

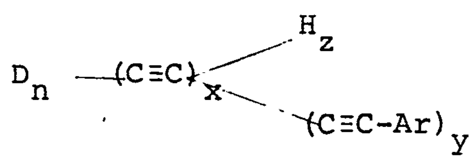

wherein D is 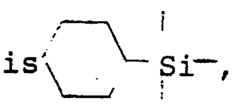, 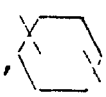, 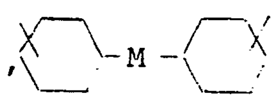 or 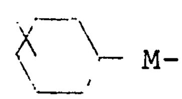,

M is -C≡C-, -C≡C-C≡C, -O-, -C(=O)-, -S-, -S(=O)-,

-Si(R)(R)-, or -O-Si(R)(R)-O, R is an alkyl or aromatic group,

Ar is an aromatic group, x is an integer greater than two but less than 30, z is either zero or an integer, y is an integer with the sum of y and z equal to x, and n is an integer from 1 to 10, said oligomer having more than two pendant acetylenic groups, the average molecular weight of said oligomer being from 200 to 10,000.

Further according to the invention there is provided a process for producing a polymerizable oligomer having the general formula:

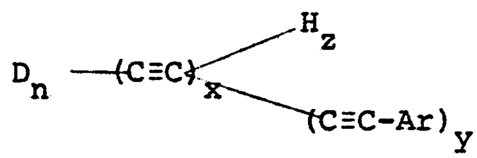

wherein D is 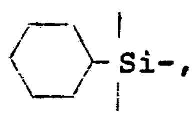, 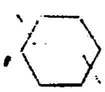, 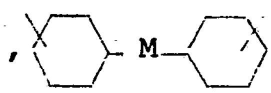 or 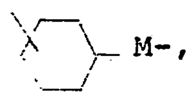,

M is $-C{\equiv}C-$, $-C{\equiv}C-C{\equiv}C$, $-O-$, $-\overset{\overset{O}{\|}}{C}-$, $-S-$, $-\overset{\overset{O}{\|}}{S}-$,

$-\underset{R}{\overset{R}{Si}}-$, or $-O-\underset{R}{\overset{R}{Si}}-O$, R is an alkyl or aromatic group;

Ar is an aromatic group, x is an integer greater than two but less than 30, z is either zero or an integer, y is an integer with the sum of y and z equal to x; and n is an integer from 1 to 10, said oligomer having more than two pendant acetylenic groups, the average molecular weight of said oligomer being from 200 to 10,000, the process comprising reacting a monomer containing at least two ethynyl groups in the presence of a catalyst with an aromatic acetylenic capping agent having a molar excess over the monomer.

Preferably the molecular weight of the oligomer is from 400 to 2,000 and at least about 60% of the acetylenic groups are diacetylenic.

When the expression "pendant" acetylenic group is used in connection with the formula above, it is used to have the ordinary meaning, i.e. to refer to groups which branch off the main chain, and also to include the terminal groups. It is preferred that diacetylenic groups constitute most of the pendant acetylenic groups.

In the U. S. Patent 3,300,457 when the starting material contained three or more terminal acetylenic groups, the product was a three-dimensional cross-linked, insoluble, infusible polymer. (See U. S. Patent 3,300,456, Column 2, lines 51-54.) Furthermore, the polymers decompose at about 180°C. (Col. 7, line 60).

The oligomers of the present invention are prepolymers which, when polymerized, undergo cross-linking and yield polymers which have many desirable and unexpected properties. The oligomers can be processed into coatings and adhesive bonds by a wide variety of coating techniques, for example, either from a Solution or from a Melt. When the prepolymers are advanced by heating above about 140°C, they yield polymers having many desirable and unexpected properties. During this heating cycle, there is essentially little or no gas evolved, which is a very desirable property for adhesives. These oligomers can also be cured by irradiation, such as ultra violet or electron beam radiation. The films are very adherent to a variety of substrates, and can be heated up to over 400°C in nitrogen with little or no loss in properties. They are very stable and moisture

resistant, and are also resistant to both acid and to base. The oligomers of the present invention may be stored, and then polymerized.

In the process of forming the oligomers of the present invention, the molecular weight of the oligomers is carefully controlled. This is done by proper choice of the amine used in the reaction, in some cases by using a reverse addition of the catalyst to the monomer, by controlling the rate of addition of the catalyst, and, most significantly, by regulating the amount of monoethynyl capping agent. Thus, in the present invention, workable, useful oligomer pre-polymers are obtained.

The capping agent referred to above is an aromatic monoethynyl compound, for example phenyl acetylene. The single ethynyl group of the capping agent enters into the coupling reaction, but because the molecule has only one ethynyl group, further coupling is stopped. It is one of the inventive features of the present invention that the molecular weight of the oligomer is controlled by using a molar excess of an aromatic monoethynyl capping agent.

The present invention requires more than two ecetylenic functions. Unless this requirement is met, the final product will not be branched and cross-linked to the desired degree.

It will be clear to those skilled in the art of high temperature polymers that the various structures indicated by D in the formula above represent our preferred structures, and that there are other aromatic

structures, such as polyester, polyimide, phenolic, etc., which also fall within the scope of the invention.

It should be noted that the oligomers of the present invention are useful when used mixed with each other. In like manner, the ingredients in D in the above formula may be mixtures of the materials specified. By polymerizing mixtures of the oligomers, useful copolymers are obtained. It should also be noted that the formula given above is intended to include those materials in which one or more substitutents are present, for example on the aromatic group shown as Ar or on one or more of the phenyl or phenylene groups. Particularly useful substitutents include alkyl groups and halogens. Such substituents do not at all enter into or interfere with the polymerization reaction. For high temperature uses, it is preferred not to have any alkyl groups because of their tending to degrade at elevated temperatures.

In the formulations of the oligomers for end use, it is to be understood that they are to be used like other prepolymers, such as phenolic and epoxies in that fillers, reinforcements and plasticisers can be incorporated over a wide range of concentrations as desired.

The following Examples are given solely for the purposes of illustration, and to demonstrate the preferred mode of carrying out the invention. They are not to be construed as limitations on the invention.

Example 1 Poly (Phenyl Triethynyl Silyl)

Synthesis

The monomer was prepared from the reaction of phenyl trichlorosilane with ethynyl magnesium bromide. The general polymerization procedure is discussed below using the above system.

A. Preparation of Poly (triethynylphenyl silane) Prepolymer I

Phenyl triethynyl silane and 300 mole % of phenylacetylene were mixed in acetone. $O_2$ was bubbled through the solution and the appropriate amount of the catalyst solution added very slowly over several hours. (Catalyst Solution: 1.0g CuCl added to stirred mixture of 0.5 ml tetramethylenediamine in 20 ml acetone. The blue green suspension was allowed to settle, and the copper-complex decanted, and stored under $N_2$. 5.0 ml solution for 0.1 mole of C≡C-H.) The reaction was allowed to proceed for 3 hours after addition was complete. The solution was poured onto 2N $H_2SO_4$, extracted, dried, and evaporated to dryness. Column chromatography with silica gel and developer hexane eliminated phenylacetylene, diphenyl diacetylene, and very low molecular weight (M.W.) material.

Reverse addition of the catalyst to the ethynyl compound instead of the prior art technique of Hay was necessary to control M.W. Both the rate of addition and % of monoethynyl capping reagent are critical to obtain desired M.W. The amount of amine complexed to the copper is always kept minimal to prevent hydrolysis due to the base.

Hydrolysis of the silyl ethynyl bond with base occurs to some extent. These materials can be removed by an alumina column chromatography step. Their presence is easily detected by Infra Red (IR) (Si-OH stretch at 3600 $cm^{-1}$) and were retained in the prepolymer preparations as a possible adhesion promoter. When kept to minimal concentrations, the loss of water with heat to form the siloxane derivative was also minimal and a thermally stable polymer results.

B. Characterization of Prepolymer

UV (Ultra Violet): Strong diacetylenic bands at 287, 304, 325 nm with weaker additional bands at 360, 410. 430 nm.

TGA (Thermogravametric Analysis): Thermally stable to 365°C with weight loss of 13%.

NMR (Nuclear Magnetic Resonance): A broad aromatic band from 6.6-7.8 $\delta$ with three maxima at 7.26, 7.44 and 7.62 $\delta$, corresponding to the split in aromatic protons.

IR: Polymer has additional ethynyl stretches between 2100-2200 $cm^{-1}$ which are not present in monomer.

The prepolymer is soluble in common organic solvents such as $CCl_4$, $CHCl_3$ and ETOAc.

Films were easily made by coating from carbon tetrachloride. Thermal treatment produced a highly cross-linked insoluble material with good electrical insulating properties.

Example 2 Poly (1,3,5- triethynyl benzene)
Prepolymer I (0-5% ethynyl content)

1,3,5- triethynel benzene 5g, (.033 moles) and 51g (0.485 moles) of phenylacetylene were mixed with 50 ml acetone and added slowly to a catalyst solution composed of 2.97g, .03 moles CuCl, 4.52 ml, .03 moles tetramethylethylene diamine in 250 ml acetone (1:1 molar ration, 5% of total C≡C-H). The catalyst solution was pre-oxidized by bubbling $O_2$ through for several minutes before the addition. The reaction mixture was stirred vigorously for the extent of the reaction, i.e. 8 hours.

The solvent was reduced to 1/2 volume and the slurry poured onto acidified methanol. (3000 ml MeOH, 20 ml conc HCl.) The precipitate was collected and washed with methanol until there was no detectable acid. The by-product diphenyl diacetylene is soluble in MeOH and is washed out. The prepolymer is dissolved in $CHCl_3$ and passed through a short silica gel (30μ mesh) column to remove insoluble/gel material. The $CHCl_3$ solution was concentrated and poured onto MeOH. The polymer was collected, dried, and yielded 11.0g of a creamy white solid.

Characterization: (Prepolymer 0-5% C≡C-H content)

LC: Liquid chromatography using a μ-$C_{18}$ reversee-phase column using 78% THF/$H_2O$ to elute.

The distinctive pattern of thirteen peaks with alternate peaks minimal or absent characterize this material.

NMR: Multiplet 7.2 - 7.6 δ no C≡C-H detectable

TGA: No weight loss until 500°C with 5% loss at 800°C under helium.

TR: See Example II. (C≡C-H stretch in this case is very small.)

M.W.: OPC 200-5000 ranges, most material having M.W. ≤ 2000 with a long tail to 8,000. VPO Mn = 557.

DSC: Two exothermic peaks with maxima at 150°C, 200°C.

UV: The optical absorption spectrum for this material has an absorption envelope from 370 nm to 200 nm with principal absorption maxima at 296 nm, 314 nm and 336 nm.

All three types of prepolymers based on poly(TEB) have similar spectrum with a slight shift to longer λ with degree of capping.

<u>Example 3</u> Poly (1,3,5- triethynyl benzene)
Prepolymer II (6-20% ethynyl content)

1,3,5-triethynylbenzene 5.0g (.033 mole) and 30.6g (0.30 mole) of phenylacetylene were mixed in 50 ml acetone and added slowly to a vigorously stirred catalyst solution consisting of 4.25g (.043 moles) CuCl and 6.93 ml, (.086 mole) pyridine (1:2 molar ratio, 10% of total ethynyl content). Only one-half of the amount of catalyst was present for the first three hours. The catalyst solution was pre-oxidized by bubbling $O_2$ through for several minutes before addition. The reaction was allowed to continue from 12-18 hours. The workup is identical to Example 2, and yielded 7.7g of creamy-white polymer.

Characterization

LC: On a μ-$C_{18}$ reverse phase liquid chromatograph column using 78% THF/$H_2O$ thirteen peaks identified by UV detection (λ=254nm) of which alternate peaks are approximately one-half the height from a gaussian distribution.

NMR: Multiplet (aromatic) 7.2-7.6 δ Singlet (C≡C-H) = 3.1 δ integrated ratio $\frac{3}{104}$ or 12% ethynyl/aromatic ring.

TGA: No weight loss 500°C, 5% weight loss at 750°C under helium.

DSC: Principal exothermic peaks 155°C, 200°C

IR: 3304cm$^{1}$ (C≡C-H), 3080cm$^{-1}$ (aromatic), 2220cm$^{-1}$ (-C≡C-) 1850cm$^{-1}$ (1.3.5 tri and mono substituted benzene), 1490cm$^{-1}$, [illegible]cm$^{-1}$ (mono substituted benzene), [illegible]cm$^{-1}$ (1.3.5 tri and mono substituted benzene)

UV: Similar to Example 2, slight increase in tail of 330 nm peak.

MW: Gel permeation chromatography using 2 x 500Å μ-styragel columns gave a molecular weight range from 200-8000 with principle components 2000 or less. Vapour Phase Osmometry: produced a Mn of 587.

This polymer, with an intermediate degree of capping, is easily coated from common organic solvents. The material polymerizes to a highly cross-linked material with heat (250°C cure for 3 hours) to an insoluble material capable of withstanding base or acid soaks. The film is highly adherent even after heating to 375°C.

Example 4 Poly (1,3,5-triethynylbenzene)-Prepolymer III (30%-80% of ethynyl content)

10g, (.067 moles) of 1,3,5triethynylbenzene and 68.3g, (.67 moles) of phenylacetylene were mixed in acetone and added slowly to a vigorously stirred catalyst solution consisting of 8.6g. (0.087 moles), CuCl and 7 ml pyridine (0.087 moles) (1:1 molar ration, 10% of total ethynyl content) in which one-half the catalyst was present for the first 3-4 hours. The catalyst was pre-oxidized before addition. The reaction was allowed to proceed for 24 hours, at which time no trace of 1,3,5-triethynyl benzene was present by TLC (thin layer chromotography).

Workup identical to Examples 2 and 3 and yielded 10.0g of light yellow polymer.

Characterization

LC: Using identical column and conditions, the thirteen peaks yield a gaussian pattern, including alternate peaks.

NMR: 7.2-7.6 δ (aromatic) 3.1 δ (ethynyl). Integrated ratio $\frac{10}{112}$ $\frac{\text{(ethynyl)}}{\text{aromatic}}$ or 40% ethynyl/ aromatic ring.

TGA: Stable to 500°C under helium.

DSC: Principle exothermic peaks 140°C, 165°C.

IR: (C≡CH) 3300cm$^{-1}$, (Aromatic) 2940cm$^{-1}$, 2220cm$^{-1}$ (Diacetylene) etc. (See Example 2) The C≡C-H absorption at 3300cm$^{-1}$ is much larger than other examples.

UV: Similar to Example 2, increase in tail of 336 nm peak.

MW: Gel permeation chromatography. Molecular range of 200-8000 with most material $\leq$ 2000.

CLAIMS

1. A polymerizable oligomer having the general formula:

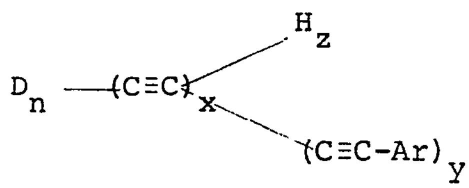

wherein D is 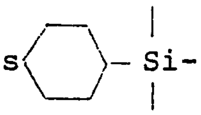, 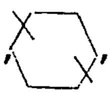, 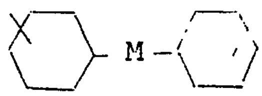 or 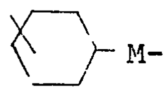,

```
                                  O        O
                                  ||       ||
M is -C≡C-, -C≡C-C≡C, -O-, -C-, -S-, -S-,

 R        R
-Si-, or -O-Si-O, R is an alkyl or aromatic group,
 R        R
```

Ar is an aromatic group, x is an integer greater than two but less than 30, z is either zero or an integer, y is an integer with the sum of y and z equal to x, and n is an integer from 1 to 10, said oligomer having more than two pendant acetylenic groups, the average molecular weight of said oligomer being from 200 to 10,000.

2. An oligomer as claimed in Claim 1, in which the molecular weight of the oligomer is from 400 to 2,000.

3. An oligomer as claimed in Claim 1 or 2, in which at least 60% of the acetylenic groups are diacetylenic.

4. A process for producing a polymerizable oligomer having the general formula:

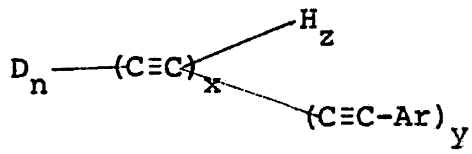

wherein D is 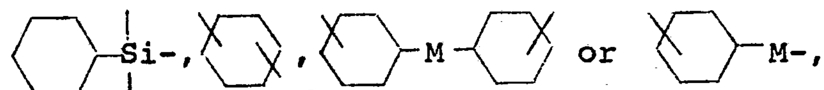 or

M is $-C\equiv C-$, $-C\equiv C-C\equiv C$, $-O-$, $-\overset{O}{\overset{\|}{C}}-$, $-S-$, $-\overset{O}{\overset{\|}{S}}-$, $-\underset{R}{\overset{R}{Si}}-$, or $-O-\underset{R}{\overset{R}{Si}}-O$, R is an alkyl or aromatic group;

Ar is an aromatic group, x is an integer greater than two but less than 30, z is either zero or an integer, y is an integer with the sum of y and z equal to x; and n is an integer from 1 to 10, said oligomer having more than two pendant acetylenic groups, the average molecular weight of said oligomer being from 200 to 10,000, the process comprising reacting a monomer containing at least two ethynyl groups in the presence of a catalyst with an aromatic acetylenic capping agent having a molar excess over the monomer.

5. A process as claimed in claim 4, in which the catalyst is a solution of cupric salt complex including an amine.

6. A process as claimed in claim 4 or 5, in which the molecular weight of the oligomer is from 400 to 2,000.

7. A process as claimed in any one of claims 4 to 6, in which at least 60% of the acetylenic groups are diacetylenic.

8. A process as claimed in any one of claims 4 to 7, in which the capping agent is phenyl acetylene.

9. A process as claimed in any one of claims 4 to 8, in which the monomer is phenyl triethynyl silane.

10. A process as claimed in any one of claims 4 to 8, in which the monomer is 1, 3, 5-triethynyl benzene.

0012798

Application number

EP 79 10 3700

European Patent Office

## PARTIAL EUROPEAN SEARCH REPORT

which under Rule 45 of the European Patent Convention shall be considered, for the purposes of subsequent proceedings, as the European search report

### DOCUMENTS CONSIDERED TO BE RELEVANT

| Category | Citation of document with indication, where appropriate, of relevant passages | Relevant to claim |
|---|---|---|
| A | CA - A - 1 034 966 (DU PONT CANADA) -- | |
| A | GB - A - 1 398 142 (HERCULES) -- | |
| A | GB -A - 1 286 817 (DOW CORNING) -- | |
| A | US - A - 3 926 897 (HERCULES) -- | |
| A | US - A - 3 627 804 (MIDLAND SILICONES) ---- | |

CLASSIFICATION OF THE APPLICATION (Int. Cl.3)

C 07 F 7/08
C 07 F 7/18
C 08 F 38/02
C 07 C 15/50
C 07 C 15/54
C 07 C 43/215
C 07 C 43/285
C 07 C 49/796
C 07 C 149/31
C 07 C 147/14

TECHNICAL FIELDS SEARCHED (Int.Cl.3)

C 07 C 15/50
C 07 F 7/08
C 08 F 38/00
C 08 F 38/02

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 9, 10

Claims searched incompletely: 1 to. 8

Claims not searched:

Reason for the limitation of the search:

The formula of the prepolymer in the claims 1 and 4 and the description give no meaningful chemical compound. The cited formula is incompatible with the examples. The search was made only on the base of the examples.

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| Berlin | 14-03-1980 | KAPTEYN |

EPO Form 1505.1 06.78